# EUROPÄISCHE PATENTSCHRIFT

(11) **EP 0 911 016 B1**
(45) Veröffentlichungstag und Bekanntmachung des Hinweises auf die Patenterteilung: **09.04.2003**
(21) Anmeldenummer: 98118851.9
(22) Anmeldetag: 06.10.1998
(51) Int. Cl.: A61K 7/00

(54) **VERWENDUNG VON ASCORBYL-2-PHOSPHORSÄUREESTERN ZUR STABILISIERUNG VON VITAMIN A UND/ODER VITAMIN A-DERIVATEN IN KOSMETISCHEN UND PHARMAZEUTISCHEN ZUBEREITUNGEN**
USE OF ASCORBYL-2-PHOSPHORIC ACID ESTERS FOR STABILISING VITAMIN A AND/OR VITAMIN A-DERIVATIVES IN COSMETIC AND PHARMACEUTIC COMPOSITIONS
UTILISATION D'ESTERS D'ASCORBYL DE L'ACIDE PHOSPHORIQUE POUR STABILISER LE VITAMINE A ET/OU LES DERIVATIVES DE VITAMINE A DANS DES COMPOSITIONS COSMETIQUE ET PHARMACEUTIQUE

(30) Priorität: 15.10.1997 DE 19745506
(43) Veröffentlichungstag der Anmeldung: 28.04.1999
(73) Patentinhaber: BASF AKTIENGESELLSCHAFT, 67056 Ludwigshafen (DE)
(72) Erfinder: Schehlmann, Volker Dr., 67354 Römerberg (DE); Westenfelder, Horst, 67435 Neustadt (DE)

(56) Entgegenhaltungen:
- EP-A- 0 440 398
- FR-A- 1 489 249
- FALBE J., REGITZ M. (HRSG.): "Römpp Chemie Lexikon (paperback)", 1995, THIEME, STUTTGART/NEW YORK

## Beschreibung

Die vorliegende Erfindung betrifft die Verwendung von Ascorbyl-2'-phosphorsäureestern zur Stabilisierung von Vitamin A und/ oder Vitamin A-Derivaten in kosmetischen und pharmazeutischen Zubereitungen.

Kosmetische und pharmazeutische Präparate mit Vitamin A und/oder Vitamin A-Derivaten gewinnen in der Dermatologie immer mehr an Bedeutung. Neben der Behandlung von Akne werden Vitamin A und Vitamin A-Derivate sehr häufig in Sonnenschutzmitteln und Präparaten gegen das sogenannte "Photoaging" eingesetzt.

Aufgrund der geringen Licht- und Oxidationsstabilität von Vitamin A und Derivaten werden an die kosmetischen bzw. pharmazeutischen Zubereitungen hohe Ansprüche hinsichtlich der Stabilisierung von Vitamin A gestellt.

Gemäß WO 93/00085, in der ebenfalls die geringe Stabilität von Retinoiden in Hautschutzmitteln beschrieben ist, werden sowohl wasser- als auch fettlösliche Antioxidantien zur Stabilisierung von Retinoiden eingesetzt. Bei den wasserlöslichen Stabilisatoren handelt es sich dabei u.a. um Ascorbinsäure sowie um Schwefelverbindungen wie z.B. Natriumsulfit, Natriumthiosulfit oder Thioglycerol. Als fettlösliche Antioxidantien werden u.a. Ascorbylpalmitat, Butylhydroxyanisol sowie α-Tocopherol verwendet.

Die o.g. Antioxidantien haben jedoch den Nachteil, daß sie, wie z.B. Ascorbinsäure oder Ascorbylpalmitat, entweder zu instabil sind oder, wie die Thio-Verbindungen, zu Geruchsbeeinträchtigungen im kosmetischen oder pharmazeutischen Produkt führen können.

Es bestand daher die Aufgabe, Stabilisatoren von Vitamin A oder Vitamin A-Derivaten für kosmetische und pharmazeutische Zubereitungen bereitzustellen, die die oben genannten Nachteile nicht aufweisen.

Überraschenderweise konnte gezeigt werden, daß die Stabilität von Vitamin A und/oder Vitamin A-Derivaten in kosmetischen und pharmazeutischen Zubereitungen durch die Verwendung von Ascorbyl-2'-phosphorsäureestern deutlich verbessert wird.

Bei den zu stabilisierenden Vitamin A-Derivaten handelt es sich beispielsweise um all-E-Retinsäure (Vitamin A-Säure), 9-Z-Retinsäure, 13-Z-Retinsäure, Retinal, Retinylester wie z.B. Retinylacetat oder Retinylpalmitat und Mischungen davon.

Die erfindungsgemäßen Ascorbyl-2'-phosphorsäureester werden bevorzugt zur Stabilisierung von besonders licht- und oxidationsempfindlichem Retinol (Vitamin A-Alkohol) verwendet.

Dabei bezieht sich die stabilisierende Wirkung sowohl auf Geruch und Farbe als auch insbesondere auf den Wirkstoffgehalt der Zubereitung.

So konnte gezeigt werden, daß besonders zur Verfärbung neigende Retinol-haltige Formulierungen in Gegenwart von Ascorbyl-2'-phosphorsäureestern sowohl bei Raumtemperatur als auch bei 45°C mindestens 3 Monate lagerstabil waren. Dabei blieben sowohl der Retinolgehalt als auch der Gehalt an Ascorbyl-2'-phosphorsäureester über den gesamten Zeitraum konstant.

Als Ascorbyl-2'-phosphorsäureester können sowohl Ascorbyl-2'-polyphosphat als auch bevorzugt Ascorbyl-2'-monophosphat und deren Salze verwendet werden. Als besonders bevorzugte Stabilisatoren sind dabei die Natrium- oder Magnesiumsalze von Ascorbyl-2'-monophosphat zu nennen.

Der Gehalt an Vitamin A in der kosmetischen oder pharmazeutischen Zubereitung liegt im Bereich von 0,01 bis 10 Gew.-%, bevorzugt im Bereich von 0,1 bis 5 Gew.-%, besonders bevorzugt im Bereich von 0,5 bis 2 Gew.-%, bezogen auf die Gesamtmenge der Zubereitung.

Der Gehalt an Ascorbyl-2'-phosphorsäureester in der kosmetischen oder pharmazeutischen Formulierung liegt im Bereich von 0,01 bis 10 Gew.-%, bevorzugt im Bereich von 0,1 bis 5 Gew.-%, besonders bevorzugt im Bereich von 0,5 bis 2 Gew.-%, bezogen auf die Gesamtmenge der Zubereitung.

Für das erfindungsgemäße Verfahren zur Stabilisierung von Vitamin A und/oder Vitamin A-Derivaten in kosmetischen und pharmazeutischen Zubereitungen können Vitamin A und/oder Vitamin A-Derivate sowie Ascorbyl-2'-phosphorsäureester den Zubereitungen sowohl separat als auch als bereits fertige Mischung vor, während oder nach ihrer Herstellung zugesetzt werden.

Von der Art des Trägers, Hilfsstoffes oder Verdünnungsmittel hängt es ab, ob die kosmetische oder pharmazeutische Zubereitung eine Lösung, ein Öl, eine Creme, eine Salbe, eine Lotion, ein Gel oder ein Schaum ist. Derartige Zubereitungen können beispielsweise der Zeitschrift "Seifen, Öle, Fette, Wachse", 1955, Seite 147 sowie Hagers Handbuch der pharmazeutischen Praxis, Springer Verlag, Band 2 (1991), Kapitel 4 entnommen werden.

Üblicherweise verwendete kosmetische Hilfsstoffe, die als Zusätze in Betracht kommen, sind beispielsweise Emulgatoren, wie Fettalkoholethoxylate, Sorbitanfettsäureester oder Lanolinderivate, Verdickungsmittel, wie Carboxymethylcellulose oder vernetzte Polyacrylsäure, Konservierungsmittel und Parfüms.

Beispiele für kosmetische Öle sind pflanzliche Öle, wie Erdnußöl, Jojobaöl, Olivenöl, Sesamöl, Baumwollsamenöl, Kokosöl, Mandelöl, Traubenkernöl, Ricinusöl oder Mineralöle, synthetische Fettsäureester und Glyceride.

Grundlage für Salben sind beispielsweise Vaseline, Lanolin, Eucerin oder Polyethylenglykole.

Grundlagen für Cremes können sowohl Emulgatoren vom Wasser-in-Öl-Typ, wie z.B. Wollfett, Sorbitanester und Monoglyceride als auch Öl-in-Wasser-Emulgatoren, wie Natriumseifen, Natriumsalze von Schwefelsäureestern einiger Fettalkohole oder Polysorbate sein.

In der kosmetischen Zubereitung können organische Lösungsmittel enthalten sein, bevorzugt wasserlösliche organische Lösungsmittel beispielsweise niedere aliphatische Alkohole wie Ethanol, Propanol oder Isopropanol, Glykole wie Ethylenglykol, Propylenglykol oder deren Polyglykole, Polyethylenglykolalkylether von C₁- bis C₄-Alkoholen, einfache oder gemischte Ketone von C₁- bis C₅-Alkoholen wie Aceton oder Methylethylketon, bevorzugt Glykole, Polyglykole oder Polyethylenglykolalkylether.

Weiterhin kann das kosmetische Mittel übliche kosmetische Zusatzstoffe enthalten, wie Farbstoffe, Parfums, Tenside, Eiweißhydrolysate, Rückfetter, Verdicker, Glanzmittel, UV-Absorber, Kräuterextrakte, Konservierungsstoffe wie bakterizide oder fungizide Stoffe, Stabilisatoren wie Magnesium- oder Aluminiumsalze von Fettsäuren, Komplexbildner wie EDTA, Antioxidatien wie BHT, BHA, Ascorbinsäure oder alpha-Tocopherol.

Die Zubereitungen, enthaltend Vitamin A und Ascorbyl-2'-phosphorsäureester, eignen sich als Mittel zum Schutz der Haut beispielsweise gegen Akne und zur Vorbeugung gegen "Photoaging".

Ferner können die Zubereitungen zum Schutz der Haare in der Haarkosmetik, beispielsweise in Haarkuren, Haarlotionen, Haarspülungen, Haaremulsionen, Spitzenfluids, Egalisierungsmitteln für Dauerwellen, "Hot-Oil-Treatment"-Präparate, Conditioner, Festigerlotionen oder Haarsprays verwendet werden.

In den nachfolgenden Beispielen werden die kosmetischen und pharmazeutischen Zubereitungen näher erläutert.

### Beispiel 1: Öl in Wasser Creme

| Massengehalt (Gew.-%) | |
|---|---|
| 2,0 | Ceteareth-6 |
| 2,0 | Ceteareth-25 |
| 10,0 | Mineral Oil. |
| 3,0 | Buxus Chinensis |
| 4,0 | Caprylic/Capric Triglyceride |
| 5,0 | Vaseline |
| 3,0 | Cetyl Stearyl Alcohol |
| 0,5 | Parabene |
| 1,0 | Natrium Ascorbyl-2'-monophosphat |
| 5,0 | Propylenglykol |
| 0,2 | EDTA |
| 0,3 | Imidazolidinylurea |
| 0,1 | Tocopherol |
| 0,5 | Tocopheryl Acetate |
| 1,0 | Retinol 10 CM (= 10 Gew.-% Retinol in Caprylic/ Capric Triglyceride) |
| ad 100 | Wasser |

### Beispiel 2: Wasser in Öl Creme

| Massengehalt (Gew.-%) | |
|---|---|
| 6,0 | PEG-7 Hydrogenated Castor Oil |
| 10,0 | Mineral Oil |
| 5,0 | Buxus Chinensis |
| 5,0 | Caprylic/Capric Triglyceride |
| 3,0 | Vaseline |
| 1,0 | Quaternium-18 Bentonite |
| 0,5 | Parabene |
| 1,0 | Natrium Ascorbyl-2'-monophosphat |
| 3,0 | 1,2-Propylenglykol |
| 0,1 | EDTA |
| 0,3 | Imidazolidinylurea |
| 0,2 | Tocopherol |
| 1,0 | Tocopheryl Acetate |
| 1,0 | Retinol 10 CM (= 10 Gew.-% Retinol in Caprylic/Capric Triglyceride) |
| ad 100 | Wasser |

### Beispiel 3: Schaumfestiger

| Massengehalt (Gew.-%) | |
|---|---|
| 2,0 | Cocotrimonium Methosulfat |
| 0,3 | Fragrance |
| 10,0 | Polyquaternium-46 |
| 2,0 | Polyquaternium-11 |
| 0,2 | Ceteareth-25 |
| 0,1 | Retinol 10 CM (= 10 Gew.-% Retinol in Caprylic/Capric Triglyceride) |
| 1,0 | Natrium Ascorbyl-2'-monophosphat |
| 1,0 | Panthenol USP |
| 15,0 | Ethanol |
| 10,0 | Propan/Butan |
| ad 100 | Wasser |

### Beispiel 4: Wasser in Öl Creme

| Massengehalt (Gew.-%) | |
|---|---|
| 3,0 | Polyglyceryl-3 Dioleate |
| 0,3 | PEG-40 Hdrogenated Castor Oil |
| 2,0 | Bees Wax |
| 8,0 | Buxus Chinensis |
| 8,0 | Mineral Oil |
| 5,0 | Cetearyl Octanoate |
| 3,0 | Hydroxyoctacosanyl Hydroxystearate |
| 3,0 | PEG-45 Dodexcyl Glycol Copolymer |
| 1,0 | Panthenol |
| 0,1 | EDTA |
| 5,0 | Glycerin |
| 0,3 | Imidazolidinylurea |
| 0,8 | Natrium Ascorbyl-2'-monophosphat |
| 1,0 | Tocopheryl Acetate |
| 0,1 | Tocopherol |
| 0,2 | Bisabolol |
| 1,0 | Retinol 10 CM (= 10 Gew.-% Retinol in Caprylic/ Capric Triglyceride) |
| 0,2 | Methyl Dibromo Glutaronitrile |
| ad 100 | Wasser |

### Beispiel 5:

### Untersuchung der Lagerstabilität der Vitamin A-Creme gemäß Beispiel 4

3 Proben der zu untersuchenden Creme wurden jeweils separat bei 6°C, 25°C und 45°C bis zu 3 Monaten aufbewahrt. Nach 6 Wochen bzw. 3 Monaten wurde jede Probe mittels HPLC auf ihren Retinol- und Natrium Ascorbyl-2'-monophosphat-Gehalt hin untersucht. Wie die Ergebnisse in Tabelle 1 zeigen, blieben sowohl der Gehalt an Retinol als auch an Natrium Ascorbyl-2'-monophosphat über den gesamten Untersuchungszeitraum hinweg konstant.

Auch wurde in keinem der drei Fälle eine Gelbfärbung der Formulierung beobachtet.

**Tabelle 1:**

| | Gehalt an Retinol [Gew.-%] | Gehalt an Na-ASMP*⁾ [Gew.-%] |
|---|---|---|
| Ausgangswert t = 0 | 0,1 (25°C) | 0,78 (25°C) |
| t = 6 Wochen | 0,08 (6°C) | 0,76 (6°C) |
| | 0,10 (25°C) | 0,80 (25°C) |
| | 0,10 (45°C) | 0,81 (45°C) |
| t = 3 Monate | 0,08 (6°C) | 0,74 (6°C) |
| | 0,09 (25°C) | 0,79 (25°C) |
| | 0,08 (45°C) | 0,77 (45°C) |

| | | |
|---|---|---|
| *⁾ Natrium Ascorbyl-2'-monophosphat | | |

## Patentansprüche

1. Verwendung von Ascorbyl-2'-phosphorsäureester zur Stabilisierung von Vitamin A und/oder Vitamin A-Derivaten in kosmetischen und pharmazeutischen Zubereitungen.

2. Verwendung von Ascorbyl-2'-phosphorsäureester nach Anspruch 1 in Konzentrationen von 0,01 bis 10 Gew.-%, bezogen auf die Gesamtmenge der kosmetischen und pharmazeutischen Zubereitungen.

3. Verwendung von Ascorbyl-2'-phosphorsäureester nach den Ansprüchen 1 und 2, **dadurch gekennzeichnet, daß** es sich um Natrium- oder Magnesiumsalze von Ascorbyl-2'-monophosphat handelt.

4. Verfahren zur Stabilisierung von Vitamin A und/oder Vitamin A-Derivaten in kosmetischen und pharmazeutischen Zubereitungen, **dadurch gekennzeichnet, daß** man Vitamin A und/oder Vitamin A-Derivate mit Ascorbyl-2'-phosphorsäureester kombiniert.

5. Verfahren nach Anspruch 4, **dadurch gekennzeichnet, daß** man Ascorbyl-2'-phosphorsäureester in Konzentrationen von 0,01 bis 10 Gew.-%, bezogen auf die Gesamtmenge der kosmetischen und pharmazeutischen Zubereitungen verwendet.

6. Verfahren nach den Ansprüchen 4 und 5, **dadurch gekennzeichnet, daß** man Vitamin A und/oder Vitamin A-Derivate mit Natrium- oder Magnesiumsalzen von Ascorbyl-2'-monophosphat kombiniert.

## Claims

1. The use of ascorbyl 2'-phosphate for stabilizing vitamin A and/or vitamin A derivatives in cosmetic and pharmaceutical preparations.

2. The use of ascorbyl 2'-phosphate as claimed in claim 1 in concentrations of from 0.01 to 10% of the total weight of the cosmetic and pharmaceutical preparations.

3. The use of ascorbyl 2'-phosphate as claimed in claims 1 and 2, which is in the form of a sodium or magnesium salt of ascorbyl 2'-monophosphate.

4. A method for stabilizing vitamin A and/or vitamin A derivatives in cosmetic and pharmaceutical preparations, which comprises combining vitamin A and/or vitamin A derivatives with ascorbyl 2'-phosphate.

5. A method as claimed in claim 4, wherein ascorbyl 2'-phosphate is used in concentrations of from 0.01 to 10% of the total weight of the cosmetic and pharmaceutical preparations.

6. A method as claimed in claims 4 and 5, wherein vitamin A and/or vitamin A derivatives are combined with sodium or magnesium salts of ascorbyl 2'-monophosphate.

## Revendications

1. Utilisation d'esters ascorbyl-2'-phosphoriques pour la stabilisation de la vitamine A et/ou de dérivés de la vitamine A dans des compositions cosmétiques et pharmaceutiques.

2. Utilisation d'esters ascorbyl-2'-phosphoriques selon la revendication 1 à des concentrations de 0,01 à 10 % du poids total des compositions cosmétiques ou pharmaceutiques.

3. Utilisation d'esters ascorbyl-2'-phosphoriques selon les revendications 1 et 2 **caractérisée par le fait qu'**il s'agit de sels de sodium ou de magnésium de l'ascorbyl-2'-monophosphate.

4. Procédé pour stabiliser la vitamine A et/ou des dérivés de la vitamine A dans des compositions cosmétiques et pharmaceutiques, **caractérisé par le fait que** l'on combine la vitamine A et/ou les dérivés de la vitamine A avec des esters ascorbyl-2'-phosphoriques.

5. Procédé selon la revendication 4, **caractérisé par le fait que** l'on utiliser les esters ascorbyl-2'-phosphoriques à des concentrations de 0,01 à 10 % du poids total de la composition cosmétique ou pharmaceutique.

6. Procédé selon les revendications 4 et 5, **caractérisé par le fait que** l'on combine la vitamine A et/ou les dérivés de la vitamine A avec des sels de sodium ou de magnésium de l'ascorbyl-2'-monophosphate.
